# EUROPEAN PATENT APPLICATION

(11) **EP 4 627 999 A1**
(43) Date of publication of application: **08.10.2025**
(21) Application number: 24217111.4
(22) Date of filing: 03.12.2024
(51) Int. Cl.: A61B 5/00, A61B 5/07, A61B 5/01, A61B 5/145

(54) **METHOD, COMPUTER PROGRAM PRODUCT, ANALYSIS DEVICE, AND SYSTEM FOR IDENTIFYING AND LOCALIZING AN UNHEALTHY GUT STATE IN A GASTROINTESTINAL TRACT**

(30) Priority: 05.04.2024 EP 24168759
(71) Applicant: Stichting IMEC Nederland, 6708 WH Wageningen (NL)
(72) Inventor: LEONARDI, Francesca, 3901 RB Veenendaal (NL); EVEN, Aniek, 6814 DT Arnhem (NL); SIJABAT, Ria, 5345 DJ OSS (NL)
(74) Representative: AWA Sweden AB

(57) **Abstract**

According to an aspect there is provided a method for identifying and localizing an unhealthy gut state in a gastrointestinal tract. The method comprises:
receiving a first time series of data representing an unhealthy gut biomarker level and a second time series of data representing pH, wherein the time series are acquired by a sensor device while the device passes through the tract;
identifying a first time point of a transition from a first to a second part of the tract based on value change of at least one of the first and second data;
analyzing at least one of the time series, for identifying occurrence of the unhealthy gut state; and
on if an occurrence of the unhealthy gut state is identified:
determining a second time point of the occurrence of unhealthy gut state; and
determining a location, along the gastrointestinal tract, of the unhealthy gut state based on the first and second time points.

## Description

### Technical field

The present disclosure relates to investigation of a gastrointestinal tract, and more specifically to a method, a computer program product, an analysis device and a system for identifying and localizing an unhealthy gut state in a gastrointestinal tract.

### Background

Gastrointestinal disorders are common and may affect for example the intestines and the stomach. For example, Irritable Bowel Syndrome (IBS) is a gastrointestinal disorder characterized by chronic abdominal pain and altered bowel habits. Several studies have correlated the persistence of mucosal inflammation with IBS and the insurgence of IBS may be associated with a subclinical inflammation which is very difficult to diagnose. Another example is Inflammatory Bowel Disease (IBD) comprising several inflammatory diseases of the intestine, of which Ulcerative Colitis (UC) and Crohn's Disease (CD) are considered the most common. UC mainly affects the large intestine, whereas CD may occur along the entire gastrointestinal tract.

Investigation of the condition of the gastrointestinal tract is challenging not least due to its limited accessibility, making collection of data and analysis difficult. Current methods include non-invasive methods as well as invasive methods. Non-invasive methods may be for example collection and analysis of stool. A problem with such non-invasive methods, however, is that they typically have low sensitivity and are often not conclusive due to the absence of reliable biomarkers. Further, with non-invasive methods it is not possible to determine the location of an unhealthy gut state, such as an inflammation. Invasive methods include for example colonoscopy. Invasive methods may be more reliable as compared to non-invasive methods, however, invasive methods are typically limited to reaching only certain portions of the gastrointestinal tract. Another drawback with invasive methods is that they are known to cause discomfort to the subject.

US 2023/0309917 discloses a method and composition for treating diseases of the gastrointestinal tract with an immunosuppressant.

In "Ingestible devices for studying the gastrointestinal physiology and their application in oral biopharmaceutics" (Advanced Drug Delivery Reviews 176 (2021)), Weitschies *et al.* discloses an ingestible sensor system for obtaining physiological data from an undisturbed gastrointestinal tract.

Hence, there is a need in the art for further improvements related to data collection and analysis of the gastrointestinal tract.

### Summary

An objective of the present disclosure is to mitigate, alleviate or eliminate one or more of the above-identified deficiencies in the art and disadvantages singly or in any combination. These and other objectives are at least partly met by the invention as defined in the independent claims. Preferred embodiments are set out in the dependent claims.

According to a first aspect there is provided a method for identifying and localizing an unhealthy gut state in a gastrointestinal tract, said method comprising:
receiving a first time series of first data representing an unhealthy gut biomarker level and a second time series of second data representing pH, wherein the first time series of first data and the second time series of second data are acquired by a sensor device while the sensor device passes through the gastrointestinal tract;
identifying a first time point of a transition from a first part of the gastrointestinal tract to a second part of the gastrointestinal tract based on a change in value of at least one of the first data in the first time series or the second data in the second time series;
analyzing at least one of the first time series of first data or the second time series of second data, for identifying occurrence of the unhealthy gut state; and
on a condition that an occurrence of the unhealthy gut state is identified:
   determining a second time point of the occurrence of the unhealthy gut state; and
   determining a location, along the gastrointestinal tract, of the occurrence of the unhealthy gut state based on the first time point and the second time point.

The first aspect is related to a method for receiving and analyzing data acquired by a sensor device while the sensor device passes through the gastrointestinal tract, to identify and localize an unhealthy gut state in a gastrointestinal tract. Thus, the method is related to processing of measurement data to determine a possible occurrence and location of the unhealthy gut state. The method may be carried out by a processing unit.

By the term "gastrointestinal tract" is here meant a gastrointestinal tract of a subject. The subject may be a human and thus the gastrointestinal tract may be part of the human body. However, it is equally conceivable that the subject is an animal with a gastrointestinal tract. Therefore, it should be realized that the method may be applicable to data acquired from any creature having a gastrointestinal tract.

By the term "sensor device" is here meant any device, unit, circuit or element configured to be ingested by the subject and to acquire at least a first time series of first data and a second time series of second data, while passing through the gastrointestinal tract of the subject. The sensor device is thus typically provided in the form of an ingestible capsule.

The unhealthy gut state is here meant any status or condition in the gastrointestinal tract deviating from a healthy state. It should be understood that the state of a gastrointestinal tract is individual, and thus defining an unhealthy gut state may be made on an individual level comparing the unhealthy gut biomarker level at different positions in the gastrointestinal tract or comparing the unhealthy gut biomarker level over time in the same subject. The unhealthy gut state may on the other hand also be compared between a subject and a reference gut state such that a deviation from the reference gut state may be defined as an unhealthy gut state.

The unhealthy gut state may be inflammation, IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, or chronic infections.

The first time series of first data represents an unhealthy gut biomarker level in the gastrointestinal tract. An example of an unhealthy gut biomarker is an inflammatory biomarker. By way of example, an inflammatory biomarker level may be low in a healthy gastrointestinal section, but may be higher at the location at which an inflammatory state occurs. By identifying a local increase in the inflammatory biomarker level, an occurrence of the inflammatory state may be identified. Thus, the time at which such an increase occurs may be determined to be the second time point.

The unhealthy gut biomarker may identify the occurrence of an unhealthy gut state, such as an inflammation. However, the unhealthy gut biomarker may as well identify the occurrence and the identity of the unhealthy gut state. Such that it may be identified as an inflammation, or as ulcers or any other unhealthy gut status.

Other examples of unhealthy gut biomarkers may be a cytokine, such as IL-6, IL-12, IL-23, or TNF-alpha. By way of further example, the inflammatory biomarker may be nitric oxide, matrix metalloproteinases, lysozyme, or hemoglobin. Further, the unhealthy gut biomarker may be pathogen- or microbiome-related biomarkers.

It serves to mention that by the term "location" is not necessarily meant an exact location, but may alternatively mean a region or part of the gastrointestinal tract. By way of example, if a standard transit time for the sensor device to pass through the gastrointestinal tract is known, an estimate of the location of the occurrence of the unhealthy gut state may be determined, for example with respect to a known time of ingestion of the sensor device.

It serves to mention that the sensor device may have different speeds in different parts of the gastrointestinal tract. Therefore, using an entry time point (ingestion of the sensor device) and an exit time point (excretion of the sensor device) alone to determine a speed of the sensor device through the gastrointestinal tract, may not provide an accurate localization of the occurrence of the unhealthy gut state. Thus, by identifying a transition from the first part to the second part of the gastrointestinal tract, along the tract and which is a known location, an improved accuracy of determining the location of the unhealthy gut state may be provided. By way of example, such a transition may be from the stomach to the small intestine, or alternatively from the small intestine to the large intestine. By way of further example, transitions between different sub-parts of the gastrointestinal tract may also be identified, such as duodenum, jejunum and ileum.

The second time series of second data represents pH in the gastrointestinal tract. At a transition from the stomach to the small intestine, an increase in pH may occur. Further, at a transition from the small intestine to the large intestine, a decrease may occur followed by a slow increase. Thus, by identifying a change in the pH, a transition from a first part to a second part of the gastrointestinal tract may be identified.

By identifying the time of occurrence of the unhealthy gut state, the second time point, in relation to the time of the transition, the first time point, e.g. between small intestine and large intestine, the localization of the unhealthy gut state may be more accurate.

The first time series of first data and the second time series of second data may be received from the sensor device in real-time while the sensor device passes through the gastrointestinal tract. This allows for the occurrence of an unhealthy gut state to be identified in real-time. However, it is equally conceivable that the analysis, and thus the identification and localization, is performed at a later stage. By way of example, the sensor device may pass through the complete gastrointestinal tract, acquiring the respective time series, and exit the gastrointestinal tract. Once the sensor device has left the gastrointestinal tract, the time series of data may be transferred to an analysis device, wirelessly or by a physical connection, after which the analysis device performs the method.

As mentioned above, the occurrence of an unhealthy gut state may be identified by a change in value of the first data in the first time series. Further, a transition from a first part to a second part of the gastrointestinal tract may be identified by a change in value of the second data in the second time series. However, it is conceivable that the occurrence of an unhealthy gut state may alternatively be identified by a change in value of the second data in the second time series. Further, it is conceivable that the transition from the first part to the second part of the gastrointestinal tract may alternatively be identified by a change in value of the first data in the first time series. As will be discussed in the following, a more accurate determination of the occurrence of the unhealthy gut state as well as the transition may be provided by analyzing both the first time series of first data and the second time series of second data.

It serves to mention that when identifying an occurrence of an unhealthy gut state in the present disclosure, no diagnosis is made, but rather an identification of the occurrence of measurement values deviant from those expected from a healthy gastrointestinal tract. However, a medical doctor provided with the data may make an assessment based on these deviant values, and thereafter make a diagnosis. Hence, the method is not a diagnostic method, but rather a method for analyzing measurement data for determining an intermediate result that may be used in diagnosis.

An advantage is that by using a combination of data of the unhealthy gut biomarker level and the pH, an improved determination of the location of the occurrence of the unhealthy gut state may be provided.

Another advantage is that it allows for identification of an unhealthy gut state at an early stage. This may in turn aid in promptly starting the correct therapy.

According to an embodiment, the first data representing the unhealthy gut biomarker level is oxidation reduction potential (ORP) data.

A healthy subject typically features an anaerobic condition in the gastrointestinal tract, specifically a healthy subject typically features an anaerobic condition in the distal part of the gastrointestinal tract. However, an unhealthy gut state associated with for example inflammation, IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, or chronic infections may increase the level of reactive oxidizing species, such as e.g. superoxide, hydroxyl, hypochlorous acid, and/or hydrogen peroxide, at the location of the unhealthy gut state. Said increase may be detected with an oxidation reduction potential (ORP) sensor. ORP is also sometimes referred to as the redox potential. ORP is measured in Volt, however the value is linked to the concentration of reducing and oxidizing species. Thus, at the location of the unhealthy gut state, a large increase in ORP values may be expected due to the presence of reactive species.

Given only as non-limiting examples, a healthy gastrointestinal tract may have an ORP value of between -500 mV and -300 mV in the large intestine. Thus, an ORP value of >-300 mV may serve as an indication of a possible occurrence of an unhealthy gut state in the large intestine. Given as further non-limiting examples, a healthy stomach may have an ORP value of between 50 mV and 250 mV while a healthy small intestine may have an ORP value of between 50 mV and -400 mV.

An advantage with this embodiment is that ORP may serve as a good marker for the unhealthy gut state.

Another advantage is that ORP may also serve as an indication of the transition between the first and second parts of the gastrointestinal tract. By way of example, the ORP value may decrease at the transition from the small intestine to the large intestine. In particular in combination with the pH, which may show a decrease followed by a slow increase at the transition from the small intestine to the large intestine, the transition may be more accurately identified.

It serves to mention that ORP may also serve as an indication of other transitions, such as from the small intestine to the large intestine, or transitions between sub-parts of the gastrointestinal tract.

It serves to mention that ORP is one way of measuring the concentration of reactive reducing and oxidizing species. However, it is conceivable that the concentration of reactive species, such as reactive oxygen species, may be measured also in other manners.

Further, the unhealthy gut biomarker is not limited to being ORP. By way of example, the unhealthy gut biomarker may be linked to inflammation and may be, but is not limited to being, a neutrophil activation marker, such as calprotectin, lactoferrin, myeloperoxidase, defensins, or S100A12. By way of further example, the inflammatory biomarker may be a cytokine, such as IL-6, IL-12, IL-23, or TNF-alpha. By way of further example, the inflammatory biomarker may be nitric oxide, matrix metalloproteinases, lysozyme, or hemoglobin. Further, the unhealthy gut biomarker may be pathogen- or microbiome-related biomarkers.

According to an embodiment, the identifying occurrence of the unhealthy gut state comprises detecting a value in the first data of the first time series, the value exceeding a threshold value.

At the occurrence of an unhealthy gut state, the first time series of first data representing the unhealthy gut biomarker level, may show an anomalous behavior compared to a healthy state. By way of example, ORP typically shows negative potential value in a healthy subject, however the ORP value may increase and approach a positive region at the location of for instance an inflammation. By way of further example, the protein calprotectin, may show a 100-fold increase in concentration at a location of an inflammation. Thus, such an increase may be clearly detectable in the first time series of first data.

It serves to mention that detecting a value of the first data exceeding the threshold value may be performed either as the sole analysis of the first and second time series, or may alternatively be performed in combination with analyzing also the second time series of second data representing pH.

An advantage with the present embodiment is that a simple yet effective manner of identification of occurrence of the unhealthy gut state may be provided.

According to an embodiment, the first part of the gastrointestinal tract is a small intestine, and wherein the second part of the gastrointestinal tract is a large intestine.

As the sensor device passes through the gastrointestinal tract it may pass one or more transitions between different parts of the gastrointestinal tract. By way of example, this may be a transition from the stomach to the small intestine of the gastrointestinal tract, or it may be a transition from the small intestine to the large intestine of the gastrointestinal tract. However, some gastrointestinal disorders, such as IBD, mainly affect the large intestine. It is therefore of interest to determine when the sensor device has transitioned from the small intestine into the large intestine. In case an occurrence of the unhealthy gut state is identified after identification of the transition from the first to the second part of the gastrointestinal tract, here meaning from the small intestine to the large intestine, it may be concluded that the occurrence of the unhealthy gut state is located in the large intestine. Dependent on how much time has passed between the first time point of the transition and the second time point of the occurrence of the unhealthy gut state, the location of the unhealthy gut state relative to the start of the large intestine may be determined.

An advantage with this embodiment is that a more accurate determination of the location of the unhealthy gut state in the large intestine may be provided.

According to an embodiment, the identifying a first time point of the transition from the first part of the gastrointestinal tract to the second part of the gastrointestinal tract is based on a decrease in value of the first data in the first time series.

Since the first time series of first data and the second time series of second data are acquired during the passage of the sensor device through the gastrointestinal tract, and since different parts of the gastrointestinal tract may have different properties with respect to the first and second data, a transition may be identified based on a change in value of at least one of the first data in the first time series or the second data in the second time series.

By way of example, in case the first part of the gastrointestinal tract is a small intestine, and wherein the second part of the gastrointestinal tract is a large intestine, the transition from the first part to the second part of the gastrointestinal tract may be identified by a decrease in value of the first data in the first time series. In other words such a transition may be identified by a decrease in the unhealthy gut biomarker level. Mentioned only as an example, such an unhealthy gut biomarker may be concentration of reactive reducing and oxidizing species, such as oxygen reactive species, which may be measured by oxidation reduction potential (ORP).

It serves to mention that the transition may also be identified by a change in the second data of the second time series, i.e. pH. When the sensor device transitions from the small intestine to the large intestine, the pH may initially show a strong decrease (in the cecum) after which the pH may slowly increase. Thus, it should be realized that by using both the first data in the first time series and the second data in the second time series when identifying the transition, the transition and thus the first time point of the transition may be identified more accurately and reliably.

An advantage with this embodiment is that the transition from the small intestine to the large intestine may show a clear decrease in the unhealthy gut biomarker level, for example in the ORP value. Such a clear decrease may easily be detectable by the sensor device.

Another advantage is that by analyzing both the first time series of first data and the second time series of second data, a more accurate and reliable determination of the transition may be provided.

According to an embodiment, the method further comprises:
receiving a third time series of third data representing temperature, wherein the third time series of third data is acquired by the sensor device while the sensor device passes through the gastrointestinal tract.

The third time series of third data may comprise information indicative of an entry time point to the gastrointestinal tract and/or an exit time point from the gastrointestinal tract. Further, the third time series of third data may comprise information indicative of a transition of the sensor device from a part of the gastrointestinal tract to another part of the gastrointestinal tract.

Alternatively, or additionally, the third time series of third data may comprise information indicative of an occurrence of the unhealthy gut state. Thus, the temperature data may be used to detect the occurrence of the unhealthy gut state. By way of example, the temperature data may show a change in value, as for example a temperature increase, at the unhealthy gut state. An advantage is that, when analyzing temperature data in combination with data of the unhealthy gut biomarker level and/or data of pH, an occurrence of the unhealthy gut state may be identified more precisely and accurately.

According to an embodiment, the method further comprises:
identifying a third time point of a transition from a third part of the gastrointestinal tract to the first part of the gastrointestinal tract based on a change in value of the second data in the second time series and absence of a change in value of the third data in the third time series.

By way of example, the first part of the gastrointestinal tract may be the small intestine of the subject. By way of further example, the third part of the gastrointestinal tract may be the stomach of the subject. Thus, the transition from the third part to the first part may be the transition from the stomach to the small intestine. It serves to mention that the duodenum is the first section of the small intestine, and thus the transition from the stomach to the small intestine is a transition from the stomach to the duodenum of the small intestine.

At the transition from the stomach to the small intestine, the pH may show an increase. This is at least partially due to the hydrochloric acid present in the stomach.

Further, a cold liquid, such as a glass of cold water, may be ingested by the subject after a first time period after the sensor device has been ingested. By way of example, the first time period may be 30 minutes. Since the liquid has a temperature lower than a body temperature of the subject, the third series of third data representing temperature is expected to show a decrease in temperature, if the sensor device is still located in the stomach at the time of the ingestion of the cold liquid. However, if the sensor device has transitioned from the stomach to the small intestine, the temperature may be unaffected by the cold liquid, since the liquid stays for a period of time in the stomach. Thus, an absence of change, or at least a negligible change, in value of the third data, in combination with an increase in pH, may be indicative of that the sensor device has transitioned from the stomach to the small intestine.

An advantage with this embodiment is that the transition from the stomach to the small intestine may be clearly identified, in the manner described above. This may further improve the accuracy of localization of an occurrence of the unhealthy gut state, especially in the stomach or the small intestine.

According to an embodiment, the determining a location of the occurrence of the unhealthy gut state comprises:
identifying an exit time point from the gastrointestinal tract based on the third data of the third time series; and
determining the location of the occurrence of the unhealthy gut state, along the second part of the gastrointestinal tract, relative to a known location of the transition from the first part of the gastrointestinal tract to the second part of the gastrointestinal tract, based on the first time point, the second time point, and the exit time point.

By the term "exit time point" is here meant the time at which the sensor device leaves the gastrointestinal tract and thus leaves the body of the subject. As long as the sensor device is inside the gastrointestinal tract, the temperature data will show the internal body temperature of the subject. In case the subject is a human, the temperature is typically around 37°C. However, upon the sensor device exiting the body it is expected to cool down to room temperature, as for example around 20°C to 25°C. Thus, by identifying a temperature decrease in the third time series, the exit time point may be determined. With the exit time point, together with the first time point of transition from the first to the second part of the gastrointestinal tract, for example from the small intestine to the large intestine, the occurrence of the unhealthy gut state may be determined relative to these known positions of the gastrointestinal tract.

By way of example, one manner in which the location of the occurrence of the unhealthy gut state may be determined, is to further determining, based on the exit time point and the first time point, a speed at which the sensor device passes through the gastrointestinal tract. The location of the occurrence of the unhealthy gut state may then be determined based on the first and second time points, and the speed.

According to an embodiment, the determining a location of the occurrence of the unhealthy gut state comprises:
identifying an entry time point from the gastrointestinal tract based on the third data of the third time series.

By the term "entry time point" is here meant the time at which the sensor device is ingested by the subject. The third time series representing temperature is expected to show a temperature increase at ingestion, typically from room temperature to the body temperature of the subject.

According to a second aspect there is provided a computer program product comprising a computer-readable storage medium storing computer-readable instructions which, when executed by a processing unit, will cause the processing unit to perform the method according to the first aspect.

According to a third aspect there is provided an analysis device for identifying and localizing an unhealthy gut state in a gastrointestinal tract, said analysis device comprising a processing unit configured to:
receiving a first time series of first data representing an unhealthy gut biomarker level and a second time series of second data representing pH, wherein the first time series of first data and the second time series of second data are acquired by a sensor device while the sensor device passes through the gastrointestinal tract;
identifying a first time point of a transition from a first part of the gastrointestinal tract to a second part of the gastrointestinal tract based on a change in value of at least one of the first data in the first time series or the second data in the second time series;
analyzing at least one of the first time series of first data or the second time series of second data, for identifying occurrence of the unhealthy gut state; and
on a condition that an occurrence of the unhealthy gut state is identified:
   determining a second time point of the occurrence of the unhealthy gut state; and
   determining a location, along the gastrointestinal tract, of the occurrence of the unhealthy gut state based on the first time point and the second time point.

The processing unit may be implemented as a general-purpose processing unit, such as a central processing unit (CPU), which may execute the instructions of one or more computer programs in order to implement the method. The processing unit may alternatively be implemented as firmware arranged in an embedded system, or as a specifically designed processing unit, such as an Application-Specific Integrated Circuit (ASIC) or a Field-Programmable Gate Array (FPGA), which may be configured to implement the method.

According to a fourth aspect there is provided a system for identifying and localizing an unhealthy gut state in a gastrointestinal tract, said system comprising:
an analysis device according to the third aspect; and
a sensor device configured to acquire the first time series of first data and the second time series of second data, while the sensor device passes through the gastrointestinal tract, and to provide the first time series of first data and the second time series of second data to the analysis device.

According to an embodiment, the sensor device further comprises an unhealthy gut biomarker sensor and a pH sensor. The unhealthy gut biomarker sensor may be configured to acquire the first time series of first data. By way of example, the unhealthy gut biomarker sensor may be and ORP sensor. The pH sensor may be configured to acquire the second time series of second data.

According to an embodiment, the sensor device may further comprise a temperature sensor.

Optionally, said sensors may be arranged onto a common electronic circuit board. The electronic circuit board may be configured to provide time series of data from the respective sensors to the analysis device.

According to an embodiment, the sensor device is provided in the form of an ingestible capsule.

An advantage is that a minimally invasive sensor device may be provided for determining unhealthy gut conditions inside and along the gastrointestinal tract yet causing minimal burden and discomfort to the subject. Further, since it is minimally invasive, it allows for investigations in the gastrointestinal tract to be performed multiple times, thereby allowing for testing the efficiency of treatments, such as anti-inflammatory treatments, if investigations are performed before as well as after treatment.

Another advantage is that a system allowing measurements to be performed directly in the gastrointestinal tract may be provided. This may provide more detailed information about the condition of the different parts of the tract.

Another advantage is that by using a combination of the unhealthy gut biomarker sensor and the pH sensor, an improved determination of the location of the occurrence of the unhealthy gut state may be provided.

Another advantage is that a relatively inexpensive system for determining unhealthy gut conditions inside and along the gastrointestinal tract may be provided, since large and expensive equipment is not required.

According to an embodiment, the sensor device may further be configured for sampling and/or drug delivery in the gastrointestinal tract. By way of example, the sensor device may be configured to take samples of the digesta, i.e. the gastrointestinal fluid. By way of further example, sampling and/or drug delivery may be performed in combination with analyzing at least one of the first time series of first data or the second time series of second data, for identifying occurrence of the unhealthy gut state in real-time. Once an occurrence of an unhealthy gut state has been identified, the sensor device may be configured to perform sampling of further data for a more detailed analysis. Alternatively, or additionally, once an occurrence of an unhealthy gut state has been identified, the sensor device may be configured to deliver a drug at the location of the unhealthy gut state.

An advantage with this embodiment is that data for a more detailed analysis of the unhealthy gut state may be provided.

Another advantage is that a sensor device that may deliver a drug locally at the location of the unhealthy gut state may be provided. Said sensor device may provide a more precise and more efficient treatment of the unhealthy gut state.

According to an embodiment, the sensor device further comprises a transmission unit configured to transmit the first time series of first data and the second time series of second data to the analysis device.

It is conceivable that the analysis device may form part of the ingestible capsule. By the present arrangement, the analysis device may be part of, or in physical connection with, the sensor device. By way of example, the time series of data may be received by an analysis device of the ingestible capsule, from the sensor device of the ingestible capsule via transmission of the time series of data by the electronic circuit board.

It is equally conceivable that the analysis device may be a device external to the ingestible capsule and thus external to the gastrointestinal tract. By way of example, the time series of data may be received by the external analysis device by wireless transmission from the sensor device or the electronic circuit board.

According to a fifth aspect there is provided a method for acquisition of data for identifying and localizing an unhealthy gut state in a gastrointestinal tract of a subject, said method comprising:
acquiring, by a sensor device being ingested by a subject and being passed through the gastrointestinal tract of the subject, a first time series of first data representing an unhealthy gut biomarker level and a second time series of second data representing pH, wherein the first time series of first data and the second time series of second data comprise information indicative of occurrence and location of the unhealthy gut state in the gastrointestinal tract.

According to an embodiment, the method further comprises:
identifying a first time point of a transition from a first part of the gastrointestinal tract to a second part of the gastrointestinal tract based on a change in value of at least one of the first data in the first time series or the second data in the second time series;
analyzing at least one of the first time series of first data or the second time series of second data, for identifying occurrence of the unhealthy gut state; and
on a condition that an occurrence of the unhealthy gut state is identified:
   determining a second time point of the occurrence of unhealthy gut state; and
   determining a location, along the gastrointestinal tract, of the occurrence of the unhealthy gut state based on the first time point and the second time point.

According to an embodiment, the method further comprises:
acquiring, by the sensor device while the sensor device passes through the gastrointestinal tract, a third time series of third data representing temperature, wherein the third time series of third data comprises information indicative of an entry time point to the gastrointestinal tract and an exit time point from the gastrointestinal tract.

According to an embodiment, the third time series of third data comprises information indicative of a transition of the sensor device from a third part of the gastrointestinal tract to the first part of the gastrointestinal tract, in relation to a liquid being ingested by the subject after a first time period after the sensor device has been ingested, the liquid having a temperature lower than a body temperature of the subject.

By way of example, the third part of the gastrointestinal tract may be the stomach, and the first part of the gastrointestinal tract may be the small intestine. Thus, upon the subject ingesting the cold liquid a temperature decrease may be identified in the third time series of third data, in case the sensor device remains in the stomach of the subject. However, in case the sensor device has transitioned from the stomach to the small intestine of the subject, the third time series of third data is expected to show no temperature decrease upon the subject ingesting the cold liquid, since the cold liquid will remain in the stomach for some time and will therefore not reach the sensor device. Thus, an absence of change, or at least a negligible change, in value of the third data, in combination with an increase in pH, may be indicative of that the sensor device has transitioned from the stomach to the small intestine.

It serves to mention that the third time series of third data may, alternatively or additionally, comprise information indicative of an occurrence of the unhealthy gut state. Put differently, the temperature data may be used to detect the occurrence of the unhealthy gut state. By way of example, the temperature data may show a change in value, such as a temperature increase, at the unhealthy gut state. This may be advantageous for example when analyzing temperature data in combination with data of the unhealthy gut biomarker level and/or data of pH, since an occurrence of the unhealthy gut state may be identified more precisely and accurately.

According to an embodiment, the method may further comprise sampling and/or drug delivery in the gastrointestinal tract. By way of example, sampling and/or drug delivery may be performed in combination with analyzing at least one of the first time series of first data or the second time series of second data, for identifying occurrence of the unhealthy gut state in real-time. Once an occurrence of an unhealthy gut state has been identified, sampling of further data may be performed for a more detailed analysis. Alternatively, or additionally, once an occurrence of an unhealthy gut state has been identified, delivery of a drug may be performed at the location of the unhealthy gut state.

An advantage with this embodiment is that data for a more detailed analysis of the unhealthy gut state may be provided.

Another advantage is that the drug may be delivered locally at the location of the unhealthy gut state, and thus a more precise and more efficient treatment of the unhealthy gut state may be provided.

Effects and features of the second, third fourth, and fifth aspects are largely analogous to those described above in connection with the first aspect. Embodiments mentioned in relation to the first aspect are largely compatible with the second, third, fourth, and fifth aspects. It is further noted that the disclosure relates to all possible combinations of features unless explicitly stated otherwise.

Other objectives, features and advantages of the present disclosure will appear from the following detailed description, from the attached claims as well as from the drawings.

### Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.
Fig. 1A schematically illustrates a system for identifying and localizing an unhealthy gut state in a gastrointestinal tract, comprising a sensor device in the form of an ingestible capsule and an external analysis device.
Fig. 1B illustrates an example of in vivo time series of data of the ORP, pH and temperature, respectively, as acquired by the sensor device when passing through the tract with an unhealthy gut state.
Fig. 1C illustrates an example of in vivo time series of data of the ORP, pH and temperature, respectively, as acquired by the sensor device when passing through the tract of a healthy subject.
Fig. 2 schematically illustrates an alternative system for identifying and localizing an unhealthy gut state in a gastrointestinal tract, comprising a sensor device and an analysis device arranged in an ingestible capsule.
Fig. 3 illustrates a schematic block diagram shortly summarizing the method for identifying and localizing an unhealthy gut state in a gastrointestinal tract.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

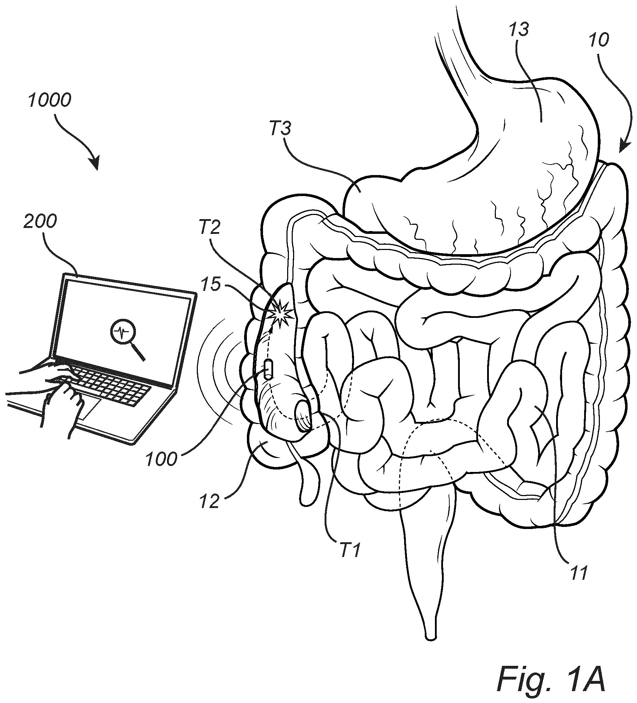

Fig. 1A schematically illustrates a system 1000 for identifying and localizing an unhealthy gut state 15 in a gastrointestinal tract 10. The unhealthy gut state may be inflammation, IBS, IBD, gastrointestinal cancer, ulcers, diabetes, ischemia, celiac disease, microbiome dysbiosis, small intestinal bacterial overgrowth (SIBO), aging, neurodegenerative diseases, cardiovascular diseases, metabolic diseases, or chronic infections.

The system 1000 comprises a sensor device 100. The sensor device 100 is provided in the form of an ingestible capsule. The sensor device 100 may thus be ingested by a subject, after which the sensor device 100 may be passed through the gastrointestinal tract 10.

The sensor device 100 comprises an unhealthy gut biomarker sensor. The unhealthy gut biomarker sensor is configured to acquire a first time series of first data representing an unhealthy gut biomarker level. In the present example, the unhealthy gut biomarker level is oxygen reduction potential (ORP). However, it should be understood that the unhealthy gut biomarker level may alternatively be any other suitable biomarker level.

The sensor device 100 further comprises a pH sensor. The pH sensor is configured to acquire a second time series of second data representing pH.

The sensor device 100 may optionally comprise a temperature sensor. The temperature sensor may be configured to acquire a third time series of third data representing temperature.

Data for the first, second and optionally third time series is acquired while the sensor device 100 passes through the gastrointestinal tract 10.

The system 1000 further comprises an analysis device 200. The analysis device 200 comprises a processing unit configured for identifying and localizing an unhealthy gut state 15 in a gastrointestinal tract 10, based on the time series of data acquired by the sensor device 100. As illustrated in Fig. 1A, the analysis device 200 may be a device external to the sensor device 100 and external to the subject. In order for the analysis device 200 to be able to analyze the data acquired by the sensor device 100, the sensor device 100 is configured to provide the first, second and optionally third time series of data to the analysis device 200. By way of example, the sensor device 100 may further comprise a transmission unit enabling the sensor device 100 to wirelessly transmit the time series of data. The wirelessly transmitted data may be received by the external analysis device 200. By way of example, the first, second and optionally third time series of data may be provided to the analysis device 200 in real-time.

A procedure to investigate the state of a gastrointestinal tract 10, including a method for acquiring data along the gastrointestinal tract 10 and a method for identifying and localizing an unhealthy gut state 15 in a gastrointestinal tract 10 of the subject, based on the acquired data, will be described in the following.

In the present example, the subject is a human. The gastrointestinal tract 10 may thus comprise a first part 11 being the small intestine, a second part 12 being the large intestine, and a third part 13 being the stomach.

The sensor device 100 may be ingested by the subject and subsequently the sensor device 100 may be passed through the gastrointestinal tract 10 of the subject. Time series of data of the unhealthy gut biomarker level, for instance an inflammatory biomarker level, e.g. ORP, pH and optionally temperature is acquired by the sensor device 100 as it passes through the tract 10. The time series of data may comprise information indicative of occurrence and location of the unhealthy gut state 15 in the gastrointestinal tract 10.

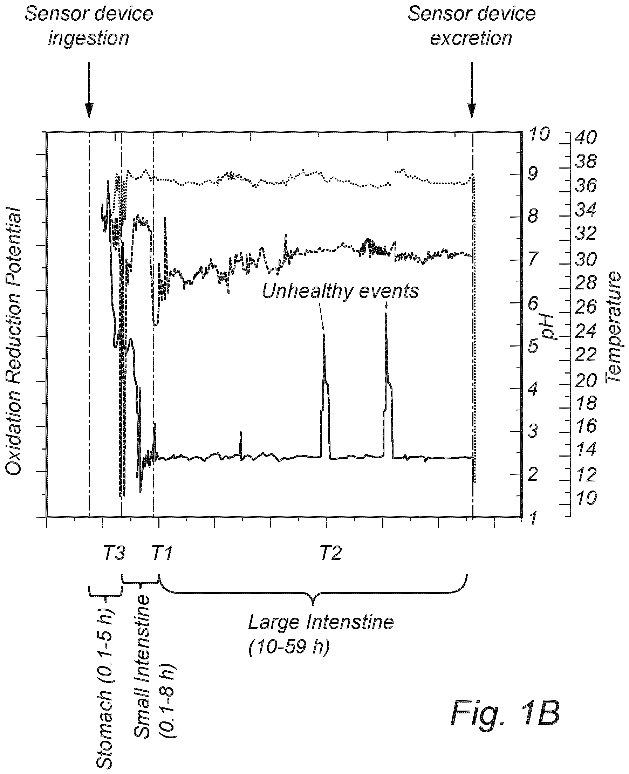

Fig. 1B illustrates an example of *in vivo* time series of data of the ORP, pH and temperature, respectively, as acquired by the sensor device 100 when passing through the tract 10.

In case the sensor device 100 of Fig. 1A comprises the optional temperature sensor, the third time series of temperature data may be indicative of the time of ingestion of the sensor device 100, i.e. the entry time point of the sensor device 100 into the gastrointestinal tract 10. Prior to ingestion, the temperature data may show a typical room temperature, as for example around 20°C to 25°C. However, upon ingestion of the sensor device 100, the temperature data is expected to increase to the internal body temperature of the subject, in the present example typically around 37°C. Thus, by the method performed by the analysis device 200, the entry time point may be determined based on the time series of temperature data.

Following the ingestion of the sensor device 100, the sensor device 100 may remain in the stomach 13 of the subject for a first time period, after which the sensor device 100 may transition from the stomach 13 to the small intestine 11. By way of example, the sensor device 100 may remain in the stomach 13 for about 30 minutes. From the time series of pH data, the transition from the stomach 13 to the small intestine 11 may be indicated by an increase in pH.

However, a confirmation that the sensor device 100 has transitioned may be provided by having the subject ingest a liquid having a temperature lower than a body temperature of the subject, after the first time period after the sensor device 100 has been ingested. In case the sensor device 100 comprises the optional temperature sensor, the time series of temperature data is expected to show negligible change as a result of the ingestion of the cold liquid, if the sensor device 100 has already transitioned from the stomach 13 to the small intestine 11, because the cold liquid will not reach the sensor device 100. Thus, an increase of value in the pH data in combination with an absence of change on temperature value, in relation to the cold liquid being ingested may be indicative of that the transition from the stomach 13 to the small intestine 11 has occurred. Based on such an occurrence in the data, the analysis device 200 may optionally identify a time point of transition from the stomach 13 to the small intestine 11, here referred to as the third time point T3. The third time point T3 of the transition is further indicated in the graph of Fig. 1B.

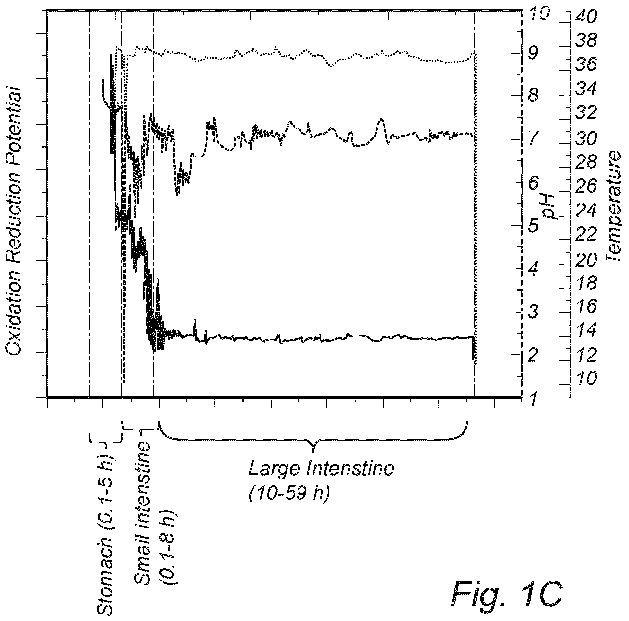

After transition, the sensor device 100 will pass through the small intestine 11 until reaching the transition from the small intestine 11 to the large intestine 12. By way of example, the transit time for the sensor device 100 to pass through the entire small intestine 11 is typically between 2 to 6 hours. A healthy subject typically features an anaerobic condition especially in the large intestine 12. Thus, at the transition from the small intestine 11 to the large intestine 12, the unhealthy gut biomarker level, i.e. the ORP in the present example, is expected to decrease. Further, in a healthy subject the ORP is expected to stay at the low level throughout the large intestine 12, as illustrated in Fig. 1C showing the ORP, pH, and temperature time series for a healthy subject. Thus, from this decrease in the ORP level, the analysis device 200 may identify the time point of the transition, here referred to the first time point T1, from the small intestine 11 to the large intestine 12.

It serves to mention that the transition may also be identified by a change in the pH data. When the sensor device 100 transitions from the small intestine 11 to the large intestine 12, the pH may initially show a strong decrease in the cecum, after which the pH may slowly increase as the sensor device 100 passes along the large intestine 12. Thus, by optionally analyzing both the first time series of ORP data and the second time series of pH data, the first time point T1 of the transition may be identified more accurately and reliably.

In the manner described above, the analysis device 200 may be able to identify at least one known location in the gastrointestinal tract 10 based on the time series of data, namely the transition between the small intestine 11 and the large intestine 12 at the first time point T1. In fact, in the present example, two known locations may be identified, thus also the transition from the stomach 13 to the small intestine 11 at the third time point T3. This provides at least one, and in this example two known locations in the gastrointestinal tract 10 relative to which the location of an occurrence of an unhealthy gut state 15 may be determined. Thus, the location of an occurrence of the unhealthy gut state 15 may be determined more accurately.

By analyzing at least one of the first time series of ORP data or the second time series of pH data, an occurrence of the unhealthy gut state 15 may be identified by the analysis device 200. As previously mentioned, a healthy subject typically features an anaerobic condition in the large intestine 12. However, an unhealthy gut state associated with for example IBS may increase the level of reactive reducing and oxidizing species, such as oxygen species at the location of the unhealthy gut state 15. Said increase may be detected with the ORP sensor. Typically, a large increase in ORP values may be expected at the unhealthy gut state 15. As illustrated in Fig. 1B, two large peaks in the ORP time series are detected in the large intestine 12, indication a possible occurrence of an unhealthy gut state 15 at each of the two locations. By way of example, the occurrence of an unhealthy gut state 15 may be identified by analysis device 200, by applying a threshold value to the time series with ORP data, and detecting if a value in the ORP data exceeds the threshold value.

When the analysis device 200 identifies an occurrence of an unhealthy gut state 15 a second time point T2 is determined, corresponding to the occurrence of the unhealthy gut state 15. In case more than one occurrence of an unhealthy gut state is identified, a time point for each of the occurrences may be determined.

Based on the first time point T1, representing the transition from the small intestine 11 to the large intestine 12, and the second time point T2, representing the occurrence of the unhealthy gut state 15, the analysis device 200 may determining the location of the occurrence of the unhealthy gut state 15, along the gastrointestinal tract 10. For example, in Fig. 1B it can be seen that the first peak indicating a possible occurrence of an unhealthy gut state is located close to the middle of the time series of the large intestine 12, and that the second peak is located in the later part of the time series. Thus, one occurrence of an unhealthy gut state 15 in the middle part of the large intestine 12 and one occurrence of an unhealthy gut state 15 in the later part of the large intestine 12 may be expected.

In case the sensor device 100 comprises the optional temperature sensor, the third time series of temperature data may be indicative of the time of excretion of the sensor device 100, i.e. the exit time point of the sensor device 100 from the gastrointestinal tract 10. The sensor device 100 inside the gastrointestinal tract may show the internal body temperature of the subject, typically around 37°C in the present example. However, upon the sensor device 100 exiting the body it is expected to cool down to room temperature, as for example around 20°C to 25°C. Thus, by identifying a temperature decrease in the third time series, the exit time point may be determined.

With the transition T1 from the small intestine 11 to the large intestine 12 and the exit time point identified by the analysis device 200, the location of the unhealthy gut state 15 at T2 may be more accurately determined. By way of example, assuming that the sensor device 100 travels with a constant speed along the large intestine 12, the time line in the time series of data may easily be translated into spatial position along the large intestine 12.

Alternatively, based on the first time point T1 and the exit time point, a speed at which the sensor device 100 passes through the large intestine 12 may be determined. A distance along the large intestine 12 from a known location of the transition of T1, to the location of the occurrence of the unhealthy gut state may be determined, based on the first time point T1, the second time point T2, and the speed.

It serves to mention that, although in the present example the occurrence of the unhealthy gut state 15 is identified only in the large intestine 12, it should be realized that occurrence of an unhealthy gut state 15 also in other parts of the gastrointestinal tract 10 may be identified in a similar manner. By way of example, an occurrence of an unhealthy gut state 15 may be determined at T2, between the transition from the stomach 13 to the small intestine 11 at T3, and the transition from the small intestine 11 to the large intestine 12 at T1.

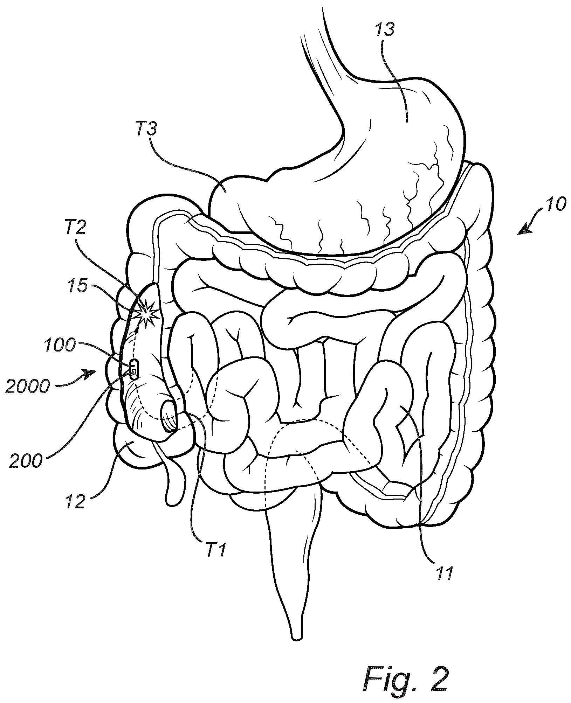

Fig. 2 schematically illustrates an alternative system 2000 for identifying and localizing an unhealthy gut state 15 in a gastrointestinal tract 10. The system 2000 comprises a sensor device 100, provided in the form of an ingestible capsule. Data for the first, second and optionally third time series is acquired while the sensor device 100 passes through the gastrointestinal tract 10. The sensor device 100 of the system 2000 shares some of the features with the sensor device 100 of the system 1000 described in relation to Fig. 1A, the details of which are not repeated here.

The ingestible capsule of the system 2000 further comprises an analysis device 200. The time series of data acquired by the sensor device 100 may be transmitted to the analysis device 200. By way of example, the time series of data may be transmitted via a physical connection to the analysis device 200. By way of further example, the time series of data may be provided to the analysis device 200 in real-time.

The analysis device 200 comprises a processing unit configured for identifying and localizing an unhealthy gut state 15 in the gastrointestinal tract 10, based on the time series of data acquired by the sensor device 100. The method for identifying and localizing an unhealthy gut state 15 in the gastrointestinal tract 10 is analogous to the method described in relation to the system 1000 of Fig. 1A, the details of which are not repeated here.

As opposed to the system 1000, wherein the method of analyzing the data is performed external to the subject, the system 2000 is configured to perform the method of analyzing the data internally in the ingestible capsule. The method may be performed in real-time such that the analysis is performed while the ingestible capsule is located inside the gastrointestinal tract 10 of the subject.

It is conceivable that the ingestible capsule may comprise a transmission unit configured to transmit data to an external unit. By way of example, the external unit may be a computer, a smart phone, a tablet, or the like. Data transmitted may be the acquired time series of data, or it may be the result of the analysis method as performed by the analysis device 200 of the ingestible capsule. By way of example, data may be presented on a screen in the form of a graph as illustrated in Figs 1B and 1C.

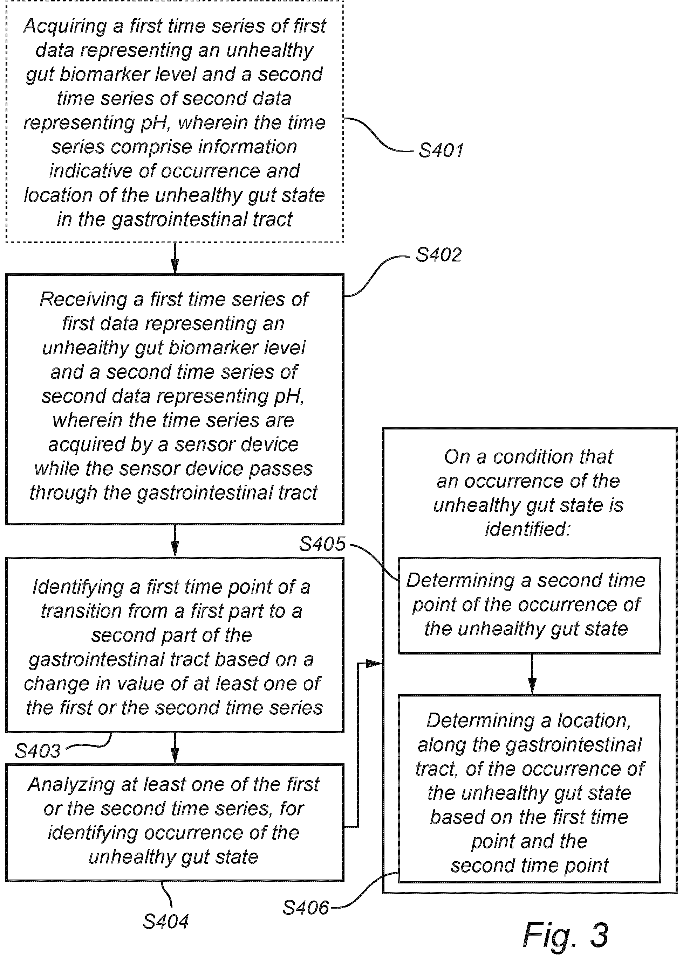

Fig. 3 illustrates a schematic block diagram shortly summarizing the method for identifying and localizing an unhealthy gut state in a gastrointestinal tract. It should be understood that the steps of the method, although listed in a specific order herein, may be performed in any order suitable.

The method may comprise acquiring S401, by a sensor device being ingested by a subject and being passed through the gastrointestinal tract of the subject, a first time series of first data representing an unhealthy gut biomarker level and a second time series of second data representing pH. The first time series of first data and the second time series of second data may comprise information indicative of occurrence and location of the unhealthy gut state in the gastrointestinal tract.

The method may comprise receiving S402 the first time series of first data representing an unhealthy gut biomarker level and the second time series of second data representing pH. The respective time series of data may be received by a processing unit, as for example a processing unit of an analysis device.

The method may comprise identifying S403 a first time point of a transition from a first part of the gastrointestinal tract to a second part of the gastrointestinal tract based on a change in value of at least one of the first data in the first time series or the second data in the second time series.

The method may comprise analyzing S404 at least one of the first time series of first data or the second time series of second data, for identifying occurrence of the unhealthy gut state.

The method may comprise, on a condition that an occurrence of the unhealthy gut state is identified, determining S405 a second time point of the occurrence of the unhealthy gut state.

The method may comprise, on a condition that an occurrence of the unhealthy gut state is identified, determining S406 a location, along the gastrointestinal tract, of the occurrence of unhealthy gut state based on the first time point and the second time point.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A method for identifying and localizing an unhealthy gut state (15) in a gastrointestinal tract (10), said method comprising:
receiving (S402) a first time series of first data representing an unhealthy gut biomarker level and a second time series of second data representing pH, wherein the first time series of first data and the second time series of second data are acquired by a sensor device (100) while the sensor device (100) passes through the gastrointestinal tract (10);
identifying (S403) a first time point (T1) of a transition from a first part (11) of the gastrointestinal tract (10) to a second part (12) of the gastrointestinal tract (10) based on a change in value of at least one of the first data in the first time series or the second data in the second time series;
analyzing (S404) at least one of the first time series of first data or the second time series of second data, for identifying occurrence of the unhealthy gut state (15); and
on a condition that an occurrence of the unhealthy gut state (15) is identified:
determining (S405) a second time point (T2) of the occurrence of the unhealthy gut state (15); and
determining (S406) a location, along the gastrointestinal tract (10), of the occurrence of the unhealthy gut state (15) based on the first time point (T1) and the second time point (T2).

2. The method according to claim 1, wherein the first data representing the unhealthy gut biomarker level is oxidation reduction potential (ORP) data.

3. The method according to any one of claims 1 or 2, wherein the identifying occurrence of the unhealthy gut state (15) comprises detecting a value in the first data of the first time series, the value exceeding a threshold value.

4. The method according to any one of the preceding claims, wherein the first part (11) of the gastrointestinal tract (10) is a small intestine, and wherein the second part (12) of the gastrointestinal tract (10) is a large intestine.

5. The method according to claim 4, wherein the identifying a first time point (T1) of the transition from the first part (11) of the gastrointestinal tract (10) to the second part (12) of the gastrointestinal tract (10) is based on a decrease in value of the first data in the first time series.

6. The method according to any one of the preceding claims, further comprising:
receiving a third time series of third data representing temperature, wherein the third time series of third data is acquired by the sensor device (100) while the sensor device (100) passes through the gastrointestinal tract (10).

7. The method according to claim 6, further comprising:
identifying a third time point (T3) of a transition from a third part (13) of the gastrointestinal tract (10) to the first part (11) of the gastrointestinal tract (10) based on a change in value of the second data in the second time series and absence of a change in value of the third data in the third time series.

8. The method according to any one of claims 6 or 7, wherein the determining a location of the occurrence of the unhealthy gut state (15) comprises:
identifying an exit time point from the gastrointestinal tract (10) based on the third data of the third time series; and
determining the location of the occurrence of the unhealthy gut state (15), along the second part (12) of the gastrointestinal tract (10), relative to a known location of the transition from the first part (11) of the gastrointestinal tract (10) to the second part (12) of the gastrointestinal tract (10), based on the first time point (T1), the second time point (T2), and the exit time point.

9. A computer program product comprising a computer-readable storage medium storing computer-readable instructions which, when executed by a processing unit, will cause the processing unit to perform the method according to any one of the preceding claims.

10. An analysis device (200) for identifying and localizing an unhealthy gut state (15) in a gastrointestinal tract (10), said analysis device (200) comprising a processing unit configured to:
receiving a first time series of first data representing an unhealthy gut biomarker level and a second time series of second data representing pH, wherein the first time series of first data and the second time series of second data are acquired by a sensor device (100) while the sensor device (100) passes through the gastrointestinal tract (10);
identifying a first time point (T1) of a transition from a first part (11) of the gastrointestinal tract (10) to a second part (12) of the gastrointestinal tract (10) based on a change in value of at least one of the first data in the first time series or the second data in the second time series;
analyzing at least one of the first time series of first data or the second time series of second data, for identifying occurrence of unhealthy gut state (15); and
on a condition that an occurrence of the unhealthy gut state (15) is identified:
determining a second time point (T2) of the occurrence of the unhealthy gut state (15); and
determining a location, along the gastrointestinal tract (10), of the occurrence of the unhealthy gut state (15) based on the first time point (T1) and the second time point (T2).

11. A system (1000, 2000) for identifying and localizing an unhealthy gut state (15) in a gastrointestinal tract (10), said system comprising:
an analysis device (200) according to claim 10; and
a sensor device (100) configured to acquire the first time series of first data and the second time series of second data, while the sensor device (100) passes through the gastrointestinal tract (10), and to provide the first time series of first data and the second time series of second data to the analysis device (200).

12. The system according to claim 11, wherein the sensor device (100) further comprises a transmission unit configured to transmit the first time series of first data and the second time series of second data to the analysis device (200).

13. A method for acquisition of data for identifying and localizing an unhealthy gut state (15) in a gastrointestinal tract (10) of a subject, said method comprising:
acquiring (S401), by a sensor device (100) being ingested by a subject and being passed through the gastrointestinal tract (10) of the subject, a first time series of first data representing an unhealthy gut biomarker level and a second time series of second data representing pH, wherein the first time series of first data and the second time series of second data comprise information indicative of occurrence and location of the unhealthy gut state (15) in the gastrointestinal tract (10).

14. The method according to claim 13, further comprising:
identifying (S403) a first time point (T1) of a transition from a first part (11) of the gastrointestinal tract (10) to a second part (12) of the gastrointestinal tract (10) based on a change in value of at least one of the first data in the first time series or the second data in the second time series;
analyzing (S404) at least one of the first time series of first data or the second time series of second data, for identifying occurrence of the unhealthy gut state (15); and
on a condition that an occurrence of the unhealthy gut state (15) is identified:
determining (S405) a second time point (T2) of the occurrence of the unhealthy gut state (15); and
determining (S406) a location, along the gastrointestinal tract (10), of the occurrence of the unhealthy gut state (15) based on the first time point (T1) and the second time point (T2).

15. The method according to any one of claims 13 or 14, further comprising:
acquiring, by the sensor device (100) while the sensor device (100) passes through the gastrointestinal tract (10), a third time series of third data representing temperature, wherein the third time series of third data comprises information indicative of an entry time point to the gastrointestinal tract (10) and an exit time point from the gastrointestinal tract (10).
